(19) Europäisches Patentamt / European Patent Office / Office européen des brevets

(11) **EP 3 278 782 A1**

(12) **EUROPEAN PATENT APPLICATION**

(43) Date of publication:
**07.02.2018   Bulletin 2018/06**

(51) Int Cl.:
*A61F 13/532* [(2006.01)]       *A61F 13/535* [(2006.01)]
*A61F 13/53* [(2006.01)]

(21) Application number: **16182344.8**

(22) Date of filing: **02.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **The Procter & Gamble Company Cincinnati, OH 45202 (US)**

(72) Inventors:
- **Messerschmidt, Andre**
  **65824 Schwalbach am Taunus (DE)**
- **Rousselange, Florian Phillip**
  **65824 Schwalbach am Taunus (DE)**

(74) Representative: **Borbach, Markus**
**Procter & Gamble Service GmbH**
**IP Department**
**Frankfurter Strasse 145**
**61476 Kronberg im Taunus (DE)**

(54) **ABSORBENT ARTICLE WITH IMPROVED FLUID STORAGE**

(57)    The invention provides an absorbent article for personal hygiene such as a diaper or training pant having a front edge and a back edge. In particular the present invention concerns, an absorbent article such as a diaper (10) or training pant, the absorbent article having a front edge (12) and a rear edge (14), a longitudinal axis extending in a longitudinal direction of the article, the article having a length L as measured along the longitudinal axis from the front edge to the back edge, the absorbent article comprising:
- an essentially liquid permeable topsheet (22),
- an essentially liquid impermeable backsheet (24),
- an absorbent core (20) between the topsheet (22) and backsheet (24),
- an acquisition-distribution system (50) which may comprise one or several layers (52, 54) and which is at least partially disposed between the absorbent core (20) and the topsheet (22),
- wherein the an absorbent core (20) comprises a first core layer (56) and a second core layer (58),
- the first core layer (56) being arranged closer to the topsheet (22) than the second core layer (58),
- the second core layer (58) extends over a length L2 from a front edge to a rear edge and over a first segment, a second segment, a third segment, a fourth segment, a fifth segment, and a sixth segment, the first segment, the second segment, the third segment, the fourth segment, the fifth segment, and the sixth segment all being of equal length, the a first segment comprising the front edge of the second core layer (58) and the sixth segment comprising the rear edge of the second core layer (58),
- the first core layer (56) comprises an orifice (70)
- the second core layer (58) comprises a superabsorbent material (62),
- the second core layer (58) comprises a first amount of superabsorbent material (62) in the first segment, a second amount of superabsorbent material (62) in the second segment, a third amount of superabsorbent material (62) in the third segment, a fourth amount of superabsorbent material (62) in the fourth segment, a fifth amount of superabsorbent material (62) in the fifth segment and a sixth amount of superabsorbent material (62) in the sixth segment,
- the fourth amount is from 0% to 80% of the fifth amount.

Fig. 1

**Description**

FIELD OF THE INVENTION

The invention provides an absorbent article for personal hygiene such as a baby diaper, a training pant or an adult incontinence product.

BACKGROUND OF THE INVENTION

Absorbent articles for personal hygiene, such as disposable diapers for infants, training pants for toddlers or adult incontinence undergarments are designed to absorb and contain body exudates, in particular large quantity of urine. A relevant absorbent article can also be provided in the form of an incontinence insert to be inserted into an undergarment. These absorbent articles comprise several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example US 5,151,092 (Buell). Absorbent articles having a core consisting essentially of SAP as absorbent material (so called "airfelt-free" cores) have also been proposed but are less common than traditional mixed cores (see e.g. WO2008/155699 (Hundorf), WO95/11652 (Tanzer), WO2012/052172 (Van Malderen)).

These absorbent articles may typically comprise leg cuffs which provide improved containment of liquids and other body exudates. Leg cuffs may also be referred to as leg bands, side flaps, barrier cuffs, or elastic cuffs. Usually each leg cuff will comprise one or more elastic string or element comprised in the chassis of the diaper for example between the topsheet and backsheet in the area of the leg openings to provide an effective seal while the diaper is in use. These elasticized elements which are substantially planar with the chassis of the absorbent article will be referred to herein as gasketing cuffs. It is also usual for the leg cuffs to comprise raised elasticized flaps, herein referred to as barrier leg cuffs, which improve the containment of fluid in the leg-torso joint regions. Each barrier leg cuff typically comprises one or more elastic strings. US 4,808,178 and US 4,909,803 (Aziz) describe disposable diapers having such raised elasticized flaps. US 4,695,278 (Lawson) and US 4,795,454 (Dragoo) describe disposable diapers having dual cuffs, including gasketing cuffs and barrier leg cuffs. US 4,704,116 (Enloe) discloses an absorbent garment comprising a pair of gasketing cuffs and a pair of barrier leg cuffs which attached to or formed from the topsheet and spaced inwardly from said elasticized leg openings, defining a waste-containment pocket.

Absorbent articles generally have a high absorbent capacity and the absorbent core can expand to several times its weight and volume. These increases will typically cause the absorbent articles to sag in the crotch region as it becomes saturated with liquid.

Although the prior art has provided different solutions to the problem of improving leakage prevention, absorbency and efficient production processes, it has still been found beneficial to develop a better core for an absorbent article for personal hygiene. In particular it has been found, that this core can be efficiently produced on commercially available equipment at high process speeds.

SUMMARY OF THE INVENTION

The invention provides an absorbent article for personal hygiene such as a diaper or training pant having a front edge and a back edge. In particular the present invention concerns, an absorbent article such as a diaper (10) or training pant, the absorbent article having a front edge (12) and a rear edge (14), a longitudinal axis extending in a longitudinal direction of the article, the article having a length L as measured along the longitudinal axis from the front edge to the back edge, the absorbent article comprising:

- an essentially liquid permeable topsheet (22),

- an essentially liquid impermeable backsheet (24),

- an absorbent core (20) between the topsheet (22) and backsheet (24),

- an acquisition-distribution system (50) which may comprise one or several layers (52, 54) and which is at least partially disposed between the absorbent core (20) and the topsheet (22),

- wherein the an absorbent core (20) comprises a first core layer (56) and a second core layer (58),

- the first core layer (56) being arranged closer to the topsheet (22) than the second core layer (58),

- the second core layer (58) extends over a length L2 from a front edge to a rear edge and over a first segment, a second segment, a third segment, a fourth segment, a fifth segment, and a sixth segment, the first segment, the second segment, the third segment, the fourth segment, the fifth segment, and the sixth segment all being of equal length, the a first segment comprising the front edge of the second core layer (58) and the sixth segment comprising the rear edge of the second core layer (58),

- the first core layer (56) comprises an orifice (70)

- the second core layer (58) comprises a superabsorbent material (62),

- the second core layer (58) comprises a first amount of superabsorbent material (62) in the first segment, a second amount of superabsorbent material (62) in the second segment, a third amount of superabsorbent material (62) in the third segment, a fourth amount of superabsorbent material (62) in the fourth segment, a fifth amount of superabsorbent material (62) in the fifth segment and a sixth amount of superabsorbent material (62) in the sixth segment,

- the fourth amount is from 0% to 80% of the fifth amount.

BRIEF DESCRIPTION OF THE DRAWINGS

Fig. 1 is a top view of an embodiment of the present invention in the form of a diaper with some layers partially removed;

Fig. 2 is a transversal cross-section of the embodiment of Fig. 1 at the crotch area;

Fig. 3 shows a top view of an embodiment of functional absorbent components of an absorbent article according to the present invention

Fig. 4 shows a sample holder useful for an IPRP analysis;

Fig. 5 shows a fluid delivery reservoir useful for an IPRP analysis.

DETAILED DESCRIPTION OF THE INVENTION

**Introduction**

As used herein, the term "absorbent article" refers to disposable devices such as infant or adult diapers, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the invention will be further illustrated in the below description and in the Figures in the form of a taped diaper. Nothing in this description should be however considered limiting the scope of the claims unless explicitly indicated otherwise. Unless indicated otherwise, the description refers to the dry article, i.e. before use and conditioned at least 24 hours at 21 °C +/- 2 °C and 50 +/- 20% Relative Humidity (RH).

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square

meter (g/m$^2$ or gsm).

The term "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element.

"Comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

**General description of the absorbent article**

In accordance with the summary above, an absorbent article according to the present invention comprises a first core layer and a second core layer and the second core layer, which is generally oriented towards the backsheet, comprises a superabsorbent material.

When a dual layer core is provided in an absorbent article, the skilled person would normally tend to avoid placing high amounts of SAP in a zone adjacent to the backsheet. There are different well known reasons for this. One reason is, that the absorbent core is typically formed on lay-down drums. The high production speeds, which typically occur when producing absorbent articles for personal hygiene, require a high influx of absorbent material in the production process and specifically in the lay-down process for the core. A high influx of particulate absorbent material, however, can lead to a considerable reduction in the life time of equipment, specifically of the lay-down drum. Therefore, the skilled person would typically choose to have higher amounts of AGM present only in those areas which are facing away from the backsheet, therefore being oriented towards the topsheet. In a dual layer core, which comprises the first layer generally adjacent to the topsheet and a second layer generally adjacent to the backsheet, the skilled person would therefore typically place AGM only in the first layer.

Further, in the context of the present invention it has been understood, that an uneven SAP distribution in the second core layer can be useful. It is useful to have an uneven SAP contribution at least in the longitudinal or lengthwise direction of the core. It is sometimes useful, to also have an uneven distribution in the lateral direction of the core. However, it can also be useful to have an even distribution in the lateral direction of the core. As said, the presence of particulate material, such as SAP material, can compromise the physical integrity of thin backsheet materials. If a high concentration of such particular material is only present in a few areas, this actual or perceived risk is only present in those areas. The risk is hardly present in areas of low concentration of SAP.

In the context of the present invention, it has been found useful to provide an orifice in the first core layer, hence in the core layer which is arranged closer to the topsheet than the second core layer. The orifice can have a variety of different shapes, for example the orifice can be rectangular or oval or circular. Often an oval shape is useful. The orifice should be large enough to receive liquid gushes. An oblong orifice, be is e.g. of oval or of rectangular or of other shape is generally useful. Such an oblong orifice has a first extension along a first, longer axis and a second extension along a second, shorter axis. It has been found useful if the longer axis is more than 5 or 10 times as long as the shorter axis.

A relatively large orifice can also be beneficial for temporarily storing liquid within the boundary of the orifice. (The second core layer can temporarily serve as a bottom defining a space for temporary storage of liquid.)

Therefore, it is useful that the orifice comprises a minimal open length of 3 mm. It can also be useful to have a minimal open length of 5, 10 or 15 mm. On the other hand, it is normally sufficient to have an orifice with a maximal open length of 200, 150, or 100 mm or less. Open length defines the length of the orifice in any direction in the plane of the first layer, be it its longest dimension or be it its shortest dimension

It has been found, that over longer usage periods there is a post insult liquid distribution between the first core layer and the second core layer. An uneven distribution in the second core layer can therefore be used to achieve a beneficial long term liquid distribution. For example, a high concentration of SAP material can be provided in areas where the storage of larger amounts of liquid does not compromise the wearing comfort of the article.

Superabsorbent particles, when they have absorbed liquids, swell. Therefore, an absorbent layer comprising such superabsorbent particles will potentially swell, and in any case, the swelling of the superabsorbent particles will reduce and avoid spaces in such layers. Without wishing to further go into any theory or without wishing to be bound by any such theory, it is generally known, that liquid transport in a layer comprising superabsorbent particles is reduced, after a first gush of liquid has been received.

These swelling effects are known to reduce the absorbent capacity of an absorbent layer to a level considerably below the capacity which in theory could be achieved by adding up the total capacity of the superabsorbent material used.

This generally known problem is particularly pertinent in multilayer cores. It also has not been sufficiently addressed for a multilayer core. While fluid distribution is handicapped *per se* due to swelling effects, fluid distribution is further handicapped if there are interfaces between different layers of the core. It therefore presents a particular challenge to fully use the capacity of a multilayer core.

Especially when a gush of liquid is received and fast liquid distribution is required, an orifice can support liquid distribution. It has been found sufficient to provide an orifice in the first core layer. An orifice can also be provided in the second core layer, however, this is normally not required.

In the context of the present invention it has been found, that dual layer cores provide improved fluid storage and handling, if the distribution of superabsorbent particles is uneven in the second core layer.

In the context of the present invention, it has been found useful to consider six segments, a first segment, a second segment, a third segment, and a fourth segment, a fifth segment, a sixth segment. The first segment is positioned adjacent to the front edge of the second core and the sixth segment is positioned adjacent to the rear edge of the absorbent core. It is often useful to provide the second segment and the fifth segment of the second core layer with a higher amount of superabsorbent material as compared to the other segments. Specifically it can be useful that the amount of superabsorbent material in the second segment and the fifth segment is higher than in the adjacent segments.

Amount herein simply refers to the mass of the super absorbent particles. Normally it is also preferred to provide a similar profile for the capacity of the super absorbent particles. Capacity refers to g/g values obtained by the Centrifuge Retention Capacity (CRC) test, as described herein below.

In one aspect, the provision of a relatively higher amount of superabsorbent particles in the second segment and in the fifth segment means a relative reduction of super absorbent particles in the third segment and in the fourth segment. Swelling effects of a nature, which can compromise fluid distribution, are therefore reduced in the third segment and in the fourth segment. This is particularly beneficial when an orifice is provided in the first core layer (which is positioned closer to the topsheet and hence above the second layer) and namely when the orifice is positioned, fully or in part, either above the third segment or above the fourth segment or above an area bridging the third segment and the fourth segment. High fluid influx over a short period of time in the area of such an orifice, can be better received by a layer which provides for a high fluid distribution in areas adjacent the orifice.

For the present invention is to considered to provide a distribution of superabsorbent material such that the fourth amount is from 0% to 80% of the fifth amount and/or such the third amount is from 0% to 80% of the second amount. It is often useful to provide a distribution of superabsorbent material such that the fourth amount is from 40% to 70% of the fifth amount and/or such the third amount is from 40% to 70% of the second amount.

If the second layer comprises a fibrous absorbent material (64) comprising the superabsorbent material (62), it can be useful to consider the ratio of the superabsorbent material (62) to the fibrous absorbent material (64), herein referred to as s/f ratio. It can be useful to provide a distribution of superabsorbent material such that the s/f ratio in the fourth segment is from 0% to 80% of the s/f ratio in the fifth segment and/or such the s/f ratio in the third segment is from 0% to 80% of the s/f ratio in the second segment. It is often useful to provide a distribution of superabsorbent material such that the s/f ratio in the fourth segment is from 40% to 70% of the s/f ratio in the fifth segment and/or such the s/f ratio in the third segment is from 40% to 70% of the s/f ratio in the second segment.

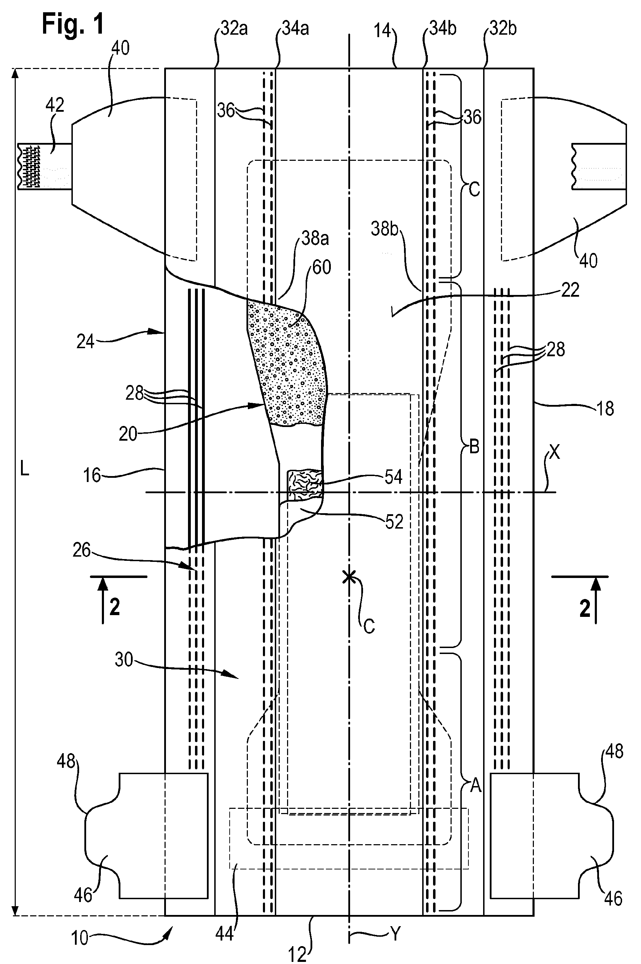

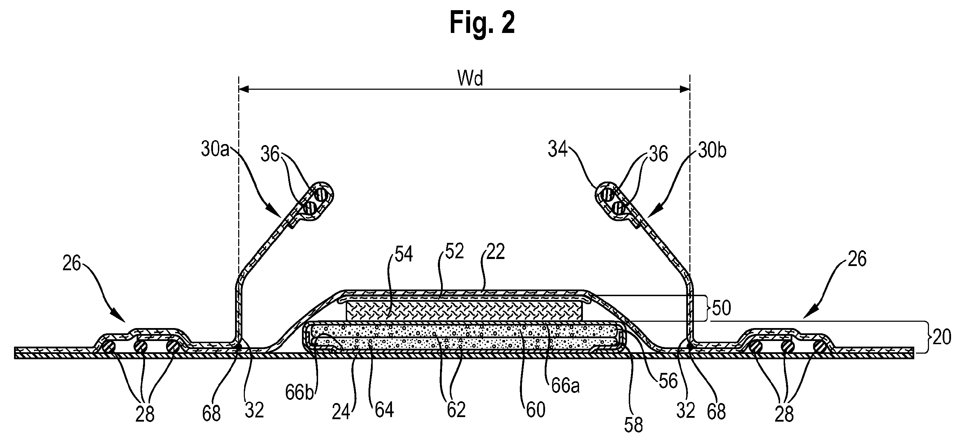

An exemplary absorbent article according to the invention in the form of an infant diaper 10 is represented in Figs. 1 and 2.

Fig. 1 is a plan view of the exemplary diaper 10, in a flattened state, with portions of the structure being cut-away to more clearly show the construction of the diaper 10. This diaper 10 is shown for illustration purpose only as the invention may be used for making a wide variety of diapers or other absorbent articles. The diaper extends from a front edge 12 to a longitudinally opposed rear edge 14. It comprises left side edge 16 and transversally opposed right side edge 18. The diaper 10 comprises an absorbent core which is positioned between topsheet 22, which is at least partially liquid permeable and backsheet 24, which is essentially impermeable to liquid.

In Fig. 1 "X" denotes a transversal access through the geometrical center of the diaper, and axis "Y" denotes the longitudinal direction. The area A denotes the front area of the diaper as seen in the longitudinal direction and C denotes the rear area of the diaper as seen in the longitudinal direction, and B denotes the central area or crotch area positioned between area A and area B, in the longitudinal direction. L denotes the length of the diaper from the front edge 12 to rear edge 14 as measured in the longitudinal direction.

The article comprises a crotch point P (represented in Fig. 1 as point C on axis Y) defined herein as the point placed on the longitudinal axis at a distance of two fifth (2/5) of L starting from the front edge 12 of the diaper 10.

The diaper 10 further comprises gasketing cuffs 26 for maintaining a tight fit of the diaper 10 to the wearer, when the diaper 10 is worn. The gasketing cuffs 26 comprise elastics 28 for maintaining the tight fit, which helps to avoid leakage.

The diaper 10 further comprises barrier leg cuffs 30 on each side of the diaper. Barrier leg cuffs comprise proximal edges 32a and 32b, which are adjacent to topsheet 22. Opposed to the respective proximal edges, the barrier leg cuffs 30 comprise distal edges 34a and 34b, respectively. In the area of the distal edges 34, further elastics 36a provided, while a portion of the distal edges 34 of the barrier leg cuffs 30 can be attached to components of the diaper 10, such as the topsheet 22, it is preferred that the barrier leg cuffs 30 also comprise unattached areas of the distal edges, herein referred to as free flaps 38. The respective free flaps 38 are typically provided in the central zone of the diaper 10.

The diaper 10 further comprises the fastening system, for fastening the diaper to the body of a wearer. This fastening system comprises two back ears 40, which comprise adhesive tapes 42. The adhesive tapes 42 can be attached to landing zone 44. In the front area, the diaper comprises front ears 46. As described below, for other embodiments other fastening systems can be useful, including mechanical fasteners and including fastening systems comprising more than two, for example for IS.

The core can optionally comprise areas, where there is a reduced amount of absorbent material or no absorbent material. These areas are referred to as channels.

Fig. 2 is transversal cross-section of the embodiment of Fig. 1 and readily shows other structural elements of the diaper. As shown in this figure, the diaper comprises an acquisition-distribution system 50. This acquisition-distribution system comprises acquisition layer 52, which first receives liquid, and distribution layer 54 underneath acquisition layer 52.

Underneath the acquisition-distribution system 50 a first core layer 56 and a second core layer 58 are positioned. The first core layer 56 is shown to comprise fibrous absorbent material 60. The first core layer 56 further comprises superabsorbent material 62, herein also referred to as superabsorbent polymer material or SAP. In the first core layer 56 superabsorbent material 62 is distributed evenly over the lengthwise direction of the core. The second core layer 58 comprises absorbent material 64, which can essentially also be fibrous absorbent material. The second core layer 58 further also comprises superabsorbent material 62, herein also referred to as superabsorbent polymer material or SAP. In the second core layer 58 superabsorbent material 62 is distributed unevenly over the lengthwise direction of the core.

Core wrap 66 wraps the first core layer 56 and the second core layer 58. In other embodiments, the first core layer 56 and the second core layer 58 could each comprise a separate core wrap. Core wrap 66 can comprise one or more components. It is shown to comprise of two components, an upper nonwoven sheet 66a and a lower nonwoven sheet 66b. The upper non-woven sheet 66a works the core in a so called C-wrap. The sheet is arranged from a C which is open towards the direction of the backsheet of the absorbent article. The respective open portion of the C, however, is covered by the lower non-woven sheet 66b. In the area of the left hand and of the right hand edge of the core, the non-woven sheet 66a and 66b overlap, as to ensure that the core is completely sealed.

The article may also comprise elasticized gasketing cuffs 26 joined to the chassis of the absorbent article, typically via the topsheet and/or backsheet, and substantially planar with the chassis of the diaper.

The Figures also show typical taped diaper components such as a fastening system comprising adhesive tabs 42 attached towards the back edge of the article and cooperating with a landing zone 44 on the front of the article. The absorbent article may also comprise other typical elements, which are not represented, such as a back elastic waist feature, a front elastic waist feature, transverse barrier cuff(s), a lotion application, etc...

The topsheet 22, the backsheet 24, the absorbent core 20 and the other article components may be assembled in a variety of well known configurations, in particular by gluing or heat embossing. Exemplary diaper configurations are described generally in US 3,860,003, US 5,221,274, US 5,554,145, US 5,569,234, US 5,580,411, and US 6,004,306. The absorbent article is preferably thin. The caliper at the crotch point P of the article may be for example from 4.0 mm to 20.0 mm, in particular from 8.0 mm to 15.0 mm, as measured with the Caliper Test described herein.

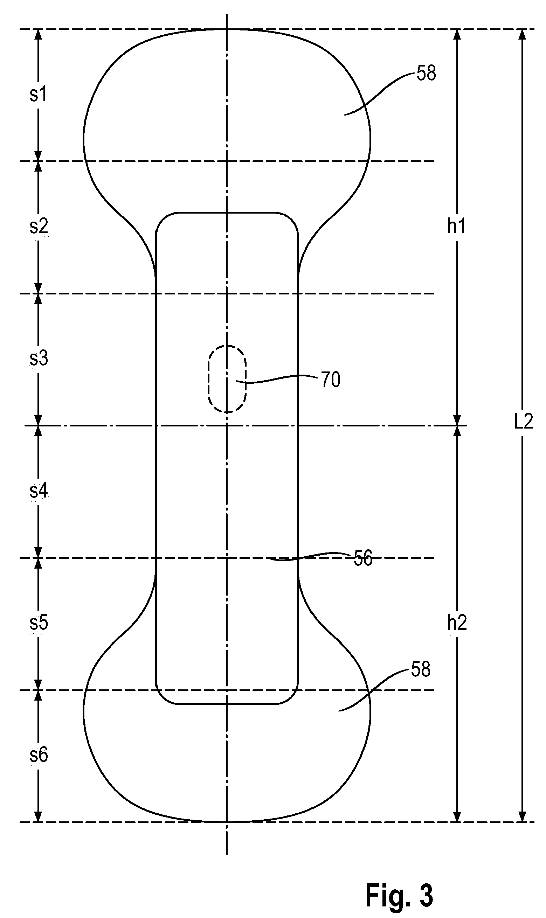

Some key aspects of the present invention will be discussed in the context of Figure 3. Figure 3 shows that an orifice (70) of oval shape provided in the first core layer (56).

It should be noted that while Figure 1 represents the general shape of the absorbent core, the shape is more accurately represented in Figure 3. Figure 1, in this regard therefore is only a general and schematic representation.

Figure 3 shows a top view onto the first core layer 56 and the second core layer 58. The second core layer 58 has the longest front to rear extension. Therefore, the length of the second core layer 58 will have most influence on the overall length of the diaper 10.

The second core layer 58 does not only have the longest longitudinal extension, but in the context of the present invention at least typically is the core layer providing most absorbent capacity. Therefore, after allowing sufficient time for fluid distribution, most fluid received by the diaper will be stored in the second absorbent layer.

The overall length of the second core layer, as indicated in Figure 3, is L2. The second core layer can be separated into segments of equal length over its longitudinal extension. As said, in the context of the present invention, it has been found useful to consider six segments, a first segment, a second segment, a third segment, and a fourth segment, a fifth segment, a sixth segment. The first segment comprises the front edge of the second core layer 58 and the sixth segment comprises the rear edge of the second core layer 58.

The length of the first segment is indicated in Figure 3 as s1, the length of the second segment is indicated as s2, the length of the third segment is indicated as s3, the length of the fourth segment is indicated as s4, the length of the fifth segment is indicated as s5, and the length of the sixth segment is indicated as s6. Moreover, the center line is indicated in Figure 3, the center line having the same distance towards the front edge of the second core layer 58 and towards the rear edge of the second core layer 58. These distances are referred to as h1 and h2, respectively.

While the length of the different segments is equal, their surface area might not be equal. While the surface area would be equal for a rectangular second core layer, the width of the core layer in the transversal direction may change, if the second core layer 58 does not have a rectangular shape.

It can be useful to provide a second core layer which has an increased surface area in the front area and in the rear area. Such a core layer is depicted in Figure 3, the shape of this core layer can be generally described as a bone-shape.

These and other components of the articles will now be discussed in yet more detail.

**Topsheet 22**

The topsheet 22 is the part of the absorbent article that is directly in contact with the wearer's skin. The topsheet 22 can be joined to the backsheet 24, the core 20 and/or any other layers as is known in the art. Usually, the topsheet 22 and the backsheet 24 are joined directly to each other in some locations (e.g. on or close to the periphery of the article) and are indirectly joined together in other locations by directly joining them to one or more other elements of the diaper 10.

The topsheet 22 is preferably compliant, soft-feeling, and non-irritating to the wearer's skin. Further, at least a portion of the topsheet 22 is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments (e.g., polyester or polypropylene or bicomponent PE/PP fibers or mixtures thereof), or combinations thereof, e.g. a combination of natural and synthetic fibers. A combination of materials can be achieved by combining at least two materials by means of needle punching, ultra-sonic bonding, ring rolling, embossing, gluing or other types of mechanical entanglement. The resulting material may maintain a dual/multiple layer structure, but may also lose a structure of distinguishable layers after such process steps. It can also be useful to provide a formed film patch underneath the topsheet.

If the topsheet 22 includes fibers, the fibers may be spunbond, carded, wet-laid, meltblown, hydroentangled, or otherwise processed as is known in the art, in particular spunbond PP nonwoven. A suitable topsheet comprising a web of staple-length polypropylene fibers is manufactured by Veratec, Inc., a Division of International Paper Company, of Walpole, MA under the designation P-8.

Suitable formed film topsheets are also described in US 3,929,135, US 4,324,246, US 4,342,314, US 4,463,045, and US 5,006,394. Other suitable topsheets may be made in accordance with US 4,609,518 and 4,629,643 issued to Curro et al. Such formed films are available from The Procter & Gamble Company of Cincinnati, Ohio as "DRI-WEAVE" and from Tredegar Corporation, based in Richmond, VA, as "CLIFF-T".

Any portion of the topsheet 22 may be coated with a lotion as is known in the art. Examples of suitable lotions include those described in US 5,607,760, US 5,609,587, US 5,643,588, US 5,968,025 and US 6,716,441. The topsheet 22 may also include or be treated with antibacterial agents, some examples of which are disclosed in PCT Publication WO95/24173. Further, the topsheet 22, the backsheet 24 or any portion of the topsheet or backsheet may be embossed and/or matte finished providing a more cloth like appearance.

The topsheet 22 may comprise one or more apertures to ease penetration of exudates therethrough, such as urine and/or feces (solid, semi-solid, or liquid). The size of at least the primary aperture is important in achieving the desired waste encapsulation performance. If the primary aperture is too small, the waste may not pass through the aperture, either due to poor alignment of the waste source and the aperture location or due to fecal masses having a diameter greater than the aperture. If the aperture is too large, the area of skin that may be contaminated by "rewet" from the article is increased. Typically, the total area of the apertures at the surface of a diaper may have an area of between about 10 mm$^2$ and about 50 mm$^2$, in particular between about 15 mm$^2$ and 35 mm$^2$. Examples of apertured topsheet are disclosed in US 6632504, assigned to BBA NONWOVENS SIMPSONVILLE. WO2011/163582 also discloses suitable colored topsheet having a basis weight of from 12 to 18 gsm and comprising a plurality of bonded points. Each of the bonded points has a surface area of from 2 mm$^2$ to 5 mm$^2$ and the cumulated surface area of the plurality of bonded points is from 10 to 25% of the total surface area of the topsheet.

Typical diaper topsheets have a basis weight of from about 10 to about 21 gsm, in particular between from about 12 to about 18 gsm but other basis weights are possible.

Backsheet24

The backsheet 24 is generally that portion of the diaper 10 positioned adjacent the garment-facing surface of the absorbent core 20 and which prevents the exudates absorbed and contained therein from soiling articles such as bed sheets and undergarments. The backsheet 24 is typically impermeable to liquids (e.g. urine). The backsheet may for example be or comprise a thin plastic film such as a thermoplastic film having a thickness of about 0.012 mm to about 0.051 mm. Exemplary backsheet films include those manufactured by Tredegar Corporation, based in Richmond, VA, and sold under the trade name CPC2 film. Other suitable backsheet materials may include breathable materials which permit vapors to escape from the diaper 10 while still preventing exudates from passing through the backsheet 24. Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, VA, and sold under the designation EXAIRE, and monolithic films such as manufactured by Clopay Corporation, Cincinnati, OH under the name HYTREL blend P18-3097. Some breathable composite materials are described in greater detail in PCT Application No. WO 95/16746 published on June 22, 1995 in the name of E. I. DuPont; US 5,938,648 to LaVon et al., US 4,681,793 to Linman et al., US 5,865,823 to Curro; and US 5,571,096 to Dobrin et al, US 6,946,585B2 to London Brown.

The backsheet 24 may be joined to the topsheet 22, the absorbent core 20 or any other element of the diaper 10 by any attachment means known in the art. Suitable attachment means are described above with respect to means for joining the topsheet 22 to other elements of the diaper 10. For example, the attachment means may include a uniform continuous layer of adhesive, a patterned layer of adhesive, or an array of separate lines, spirals, or spots of adhesive. Suitable attachment means comprises an open pattern network of filaments of adhesive as disclosed in US 4,573,986. Other suitable attachment means include several lines of adhesive filaments which are swirled into a spiral pattern, as is illustrated by the apparatus and methods shown in US 3,911,173, US 4,785,996; and US 4,842,666. Adhesives which have been found to be satisfactory are manufactured by H. B. Fuller Company of St. Paul, Minnesota and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

## Absorbent core 20

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

The absorbent core 20 of the invention comprises a first core layer 56 and a second core layer 58. As explained, the absorbent article might comprise and acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to allow liquid or gases to pass through it. Hence, the layers of the acquisition distribution system should generally be more permeable than the layers of the core system, as these layers are meant to distribute liquid to the absorbent core, where the liquid is ultimately stored.

Therefore, the absorbent core layers and the absorbent core in accordance with the present invention will have an IPRP value, determined in accordance with the IPRP analysis described herein below, which is less than 1200. Useful core layers will have a permeability which is less than 1000, less than 800, or less than 600. Especially a core layer which comprises a combination of superabsorbent materials and other absorbent materials, for example airfelt materials, can also achieve values considerably lower than that, useful layers have been found to have values of less than 500, or 300 or even 100.

Core layers with a relatively low permeability store liquids more reliably, also under pressure, which reduces the risk of leakage from the core and thereby the absorbent article.

On the other hand, it has been found that layers useful for the acquisition distribution system can have much higher IPRPs values, as described herein below.

The absorbent core can comprise absorbent material with a varying amount of superabsorbent polymers (herein abbreviated as "SAP"), often enclosed within a core wrap. The SAP content can represent from 0% to 80% by weight of the absorbent material contained in the core wrap. Often an SAP content of 10% to 50% or 20% to 40% by weight of the absorbent material contained in the core wrap is useful. The core wrap is not considered as absorbent material for the purpose of assessing the percentage of SAP in the absorbent core.

By "absorbent material" it is meant a material which has some absorbency property or liquid retaining properties, such as SAP, cellulosic fibers as well as synthetic fibers. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material. Herein, absorbent materials in form of SAP particles in the shape of fibers, which can also be referred to as superabsorbent fibers, are not understood as fibrous absorbent materials, but as SAP.

The SAP content may be higher than 30%, for example at least 40%, at least 50%, at least 80% of the weight of the absorbent material contained within the core wrap. The absorbent material may in particular comprise from 10 to 70, for example 30 to 60 weight percent of natural or synthetic fibers.

The absorbent core may comprise a generally planar top edge and a generally planar bottom edge. In some embodiments, the absorbent material will be advantageously distributed in higher amount towards the front edge than towards the rear edge as more absorbency is required at the front. In other embodiments, typically embodiments for other uses of an absorbent article, such as care of elderly incontinent people versus care of babies, the absorbent material will be advantageously distributed in higher amount towards the rear edge than towards the front edge as more absorbency is required at the rear area.

According to the present invention, the core comprises a fibrous absorbent material comprising superabsorbent material. It is useful that superabsorbent material is distributed across the fibrous material. For example, the fibrous absorbent material can provide a matrix for the superabsorbent material, which is thereby mechanically stabilized and held in an essentially uniform distribution over the lateral and transversal direction of the core. Superabsorbent material is normally not comprised in single fibers of the (non-superabsorbent) fibrous material.

The core wrap may be formed by two separate sheets of nonwoven material which may be at least partially sealed along the edges of the absorbent core. The core wrap may be at least partially sealed along its front edge, back edge and two longitudinal edges so that substantially no absorbent material leaks out of the absorbent core wrap.

The absorbent core of the invention may further comprise adhesive for example to help immobilizing the SAP within the core wrap and/or to ensure integrity of the core wrap, in particular when the core wrap is made of two or more substrates. Such an adhesive can be provided in the form of fibrous thermoplastic adhesive material.

The optional core wrap will typically extend to a larger area than strictly needed for containing the absorbent material within. The absorbent core advantageously achieve an SAP loss of no more than about 70%, 60%, 50%, 40%, 30%, 20%, 10% according to the Wet Immobilization Test described in WO2010/0051166A1.

Cores comprising relatively high amount of SAP with various core designs have been proposed in the past, see for example in US 5,599,335 (Goldman), EP1,447,066 (Busam), WO95/11652 (Tanzer), US 2008/0312622A1 (Hundorf), WO2012/052172 (Van Malderen). The present core construction allows also for a lesser amount of SAP.

The superabsorbent material may be a continuous layer present within the core wrap. In other embodiments, the absorbent material may be comprised of individual pockets or stripes of absorbent material enclosed within the core wrap.

The fibrous thermoplastic adhesive material may be at least partially in contact with the superabsorbent material in the land areas and at least partially in contact with the substrate layer in the junction areas. This imparts an essentially three-dimensional structure to the fibrous layer of thermoplastic adhesive material, which in itself is essentially a two-dimensional structure of relatively small thickness, as compared to the dimension in length and width directions. Thereby, the fibrous thermoplastic adhesive material may provide cavities to cover the absorbent material in the land area, and thereby immobilizes this absorbent material.

The thermoplastic adhesive material may comprise, in its entirety, a single thermoplastic polymer or a blend of thermoplastic polymers, having a softening point, as determined by the ASTM Method D-36-95 "Ring and Ball", in the range between 50°C and 300°C, and/or the thermoplastic adhesive material may be a hotmelt adhesive comprising at least one thermoplastic polymer in combination with other thermoplastic diluents such as tackifying resins, plasticizers and additives such as antioxidants.

The thermoplastic polymer has typically a molecular weight (Mw) of more than 10,000 and a glass transition temperature (Tg) usually below room temperature or -6 °C < Tg < 16 °C. Typical concentrations of the polymer in a hotmelt are in the range of about 20 to about 40% by weight. The thermoplastic polymers may be water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof. Other suitable thermoplastic polymers that may be employed are metallocene polyolefins, which are ethylene polymers prepared using single-site or metallocene catalysts. Therein, at least one comonomer can be polymerized with ethylene to make a copolymer, terpolymer or higher order polymer. Also applicable are amorphous polyolefins or amorphous polyalphaolefins (APAO) which are homopolymers, copolymers or terpolymers of C2 to C8 alpha olefins.

The tackifying resin may exemplarily have a Mw below 5,000 and a Tg usually above room temperature, typical concentrations of the resin in a hotmelt are in the range of about 30 to about 60%, and the plasticizer has a low Mw of typically less than 1,000 and a Tg below room temperature, with a typical concentration of about 0 to about 15%.

The thermoplastic adhesive used for the fibrous (thermoplastic adhesive) layer preferably has elastomeric properties, such that the web formed by the fibers on the SAP layer is able to be stretched as the SAP swell. Exemplary elastomeric, hotmelt adhesives include thermoplastic elastomers such as ethylene vinyl acetates, polyurethanes, polyolefin blends of a hard component (generally a crystalline polyolefin such as polypropylene or polyethylene) and a Soft component (such as ethylenepropylene rubber); copolyesters such as poly (ethylene terephthalate-co-ethylene azelate); and thermoplastic elastomeric block copolymers having thermoplastic end blocks and rubbery mid blocks designated as A-B-A block copolymers: mixtures of structurally different homopolymers or copolymers, e.g., a mixture of polyethylene or polystyrene with an A-B-A block copolymer; mixtures of a thermoplastic elastomer and a low molecular weight resin modifier, e.g., a mixture of a styrene-isoprenestyrene block copolymer with polystyrene; and the elastomeric, hot-melt, pressure-sensitive adhesives described herein. Elastomeric, hot-melt adhesives of these types are described in more detail in U.S. Patent No. 4,731,066 issued to Korpman on Mar. 15, 1988.

The thermoplastic adhesive material is advantageously applied as fibers. The fibers may exemplarily have an average thickness of about 1 to about 50 micrometers or about 1 to about 35 micrometers and an average length of about 5 mm to about 50 mm or about 5mm to about 30 mm. To improve the adhesion of the thermoplastic adhesive material to the substrate or to any other layer, in particular any other nonwoven layer, such layers may be pre-treated with an auxiliary adhesive. The fibers adhere to each other to form a fibrous layer, which can also be described as a mesh.

In certain embodiments, the thermoplastic adhesive material will meet at least one, or several, or all of the following parameters. An exemplary thermoplastic adhesive material may have a storage modulus G' measured at 20°C of at least 30,000 Pa and less than 300,000 Pa, or less than 200,000 Pa, or between 140,000 Pa and 200,000 Pa, or less than 100,000 Pa. In a further aspect, the storage modulus G' measured at 35°C may be greater than 80,000 Pa. In a further aspect, the storage modulus G' measured at 60°C may be less than 300,000 Pa and more than 18,000 Pa, or more than 24,000 Pa, or more than 30,000 Pa, or more than 90,000 Pa. In a further aspect, the storage modulus G' measured at 90°C may be less than 200,000 Pa and more than 10,000 Pa, or more than 20,000 Pa, or more then 30,000 Pa. The storage modulus measured at 60°C and 90°C may be a measure for the form stability of the thermoplastic adhesive material at elevated ambient temperatures. This value is particularly important if the absorbent product is used in a hot climate where the thermoplastic adhesive material would lose its integrity if the storage modulus G' at 60 °C and 90 °C is not sufficiently high.

G' can be measured using a rheometer as indicated in WO2010/27719. The rheometer is capable of applying a shear stress to the adhesive and measuring the resulting strain (shear deformation) response at constant temperature. The adhesive is placed between a Peltier-element acting as lower, fixed plate and an upper plate with a radius R of e.g., 10 mm, which is connected to the drive shaft of a motor to generate the shear stress. The gap between both plates has a height H of e.g., 1500 micron. The Peltier- element enables temperature control of the material (+0.5°C). The strain rate and frequency should be chosen such that all measurements are made in the linear viscoelastic region.

## Superabsorbent polymer (SAP)

Superabsorbent material, herein also referred to as superabsorbent polymer material, superabsorbent polymers or SAP, refers to absorbent materials which are cross-linked polymeric materials that can absorb at least 10 times their weight of an aqueous 0.9% saline solution as measured using the Centrifuge Retention Capacity (CRC) test (EDANA method WSP 241.2-05E). The SAP used may in particular have a CRC value of more than 20 g/g, or more than 24 g/g, or of from 20 to 50 g/g, or from 20 to 40 g/g, or 24 to 30 g/g. The SAP useful in the present invention include a variety of water-insoluble, but water-swellable polymers capable of absorbing large quantities of fluids.

The superabsorbent polymer can be in particulate form so as to be flowable in the dry state. Typical particulate absorbent polymer materials are made of poly(meth)acrylic acid polymers. However, e.g. starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The superabsorbent polymer may be polyacrylates and polyacrylic acid polymers that are internally and/ or surface cross-linked. Suitable materials are described in the PCT Patent Application WO07/047598 or for example WO07/046052 or for example WO2009/155265 and WO2009/155264. In some embodiments, suitable superabsorbent polymer particles may be obtained by current state of the art production processes as is more particularly as described in WO 2006/083584. The superabsorbent polymers are preferably internally cross-linked, i.e. the polymerization is carried out in the presence of compounds having two or more polymerizable groups which can be free-radically copolymerized into the polymer network. Useful crosslinkers include for example ethylene glycol dimethacrylate, diethylene glycol diacrylate, allyl methacrylate, trimethylolpropane triacrylate, triallylamine, tetraallyloxyethane as described in EP-A 530 438, di- and triacrylates as described in EP-A 547 847, EP-A 559 476, EP-A 632 068, WO 93/21237, WO 03/104299, WO 03/104300, WO 03/104301 and in DE-A 103 31 450, mixed acrylates which, as well as acrylate groups, include further ethylenically unsaturated groups, as described in DE-A 103 31 456 and DE-A 103 55 401, or crosslinker mixtures as described for example in DE-A 195 43 368, DE-A 196 46 484, WO 90/15830 and WO 02/32962 as well as cross-linkers described in WO2009/155265. The superabsorbent polymer particles may be externally surface cross-linked, or: post cross-linked). Useful post-crosslinkers include compounds including two or more groups capable of forming covalent bonds with the carboxylate groups of the polymers. Useful compounds include for example alkoxysilyl compounds, polyaziridines, polyamines, polyamidoamines, di- or polyglycidyl compounds as described in EP-A 083 022, EP-A 543 303 and EP-A 937 736, polyhydric alcohols as described in DE-C 33 14 019, cyclic carbonates as described in DE-A 40 20 780, 2-oxazolidone and its derivatives, such as N-(2-hydroxyethyl)-2-oxazolidone as described in DE-A 198 07 502, bis- and poly-2-oxazolidones as described in DE-A 198 07 992, 2-oxotetrahydro-1,3-oxazine and its derivatives as described in DE-A 198 54 573, N-acyl-2-oxazolidones as described in DE-A 198 54 574, cyclic ureas as described in DE-A 102 04 937, bicyclic amide acetals as described in DE-A 103 34 584, oxetane and cyclic ureas as described in EP1,199,327 and morpholine-2,3-dione and its derivatives as described in WO03/031482.

In some embodiments, the SAP are formed from polyacrylic acid polymers/ polyacrylate polymers, for example having a neutralization degree of from 60% to 90%, or about 75%, having for example sodium counter ions.

The SAP useful for the present invention may be of numerous shapes. The term "particles" refers to granules, fibers, flakes, spheres, powders, platelets and other shapes and forms known to persons skilled in the art of superabsorbent polymer particles. In some embodiments, the SAP particles can be in the shape of fibers, i.e. elongated, acicular superabsorbent polymer particles. In those embodiments, the superabsorbent polymer particles fibers have a minor dimension (i.e. diameter of the fiber) of less than about 1 mm, usually less than about 500 $\mu$m, and preferably less than 250 $\mu$m down to 50 $\mu$m. The length of the fibers is preferably about 3 mm to about 100 mm. The fibers can also be in the form of a long filament that can be woven.

Typically, SAP are spherical-like particles. In contrast to fibers, "spherical-like particles" have a longest and a smallest dimension with a particulate ratio of longest to smallest particle dimension in the range of 1-5, where a value of 1 would equate a perfectly spherical particle and 5 would allow for some deviation from such a spherical particle. The superabsorbent polymer particles may have a particle size of less than 850 $\mu$m, or from 50 to 850 $\mu$m, preferably from 100 to 710 $\mu$m, more preferably from 150 to 650 $\mu$m, as measured according to EDANA method WSP 220.2-05. SAP having a relatively low particle size help to increase the surface area of the absorbent material which is in contact with liquid exudates and therefore support fast absorption of liquid exudates.

The SAP may have a particle sizes in the range from 45 $\mu$m to 4000 $\mu$m, more specifically a particle size distribution within the range of from 45 $\mu$m to about 2000 $\mu$m, or from about 100 $\mu$m to about 1000, 850 or 600 $\mu$m. The particle size distribution of a material in particulate form can be determined as it is known in the art, for example by means of dry sieve analysis (EDANA 420.02 "Particle Size distribution).

In some embodiments herein, the superabsorbent material is in the form of particles with a mass medium particle size up to 2 mm, or between 50 microns and 2 mm or to 1 mm, or preferably from 100 or 200 or 300 or 400 or 500 $\mu$m, or to 1000 or to 800 or to 700 $\mu$m; as can for example be measured by the method set out in for example EP-A-0,691,133. In some embodiments of the invention, the superabsorbent polymer material is in the form of particles whereof at least 80% by weight are particles of a size between 50 $\mu$m and 1200 $\mu$m and having a mass median particle size between any of the range combinations above. In addition, or in another embodiment of the invention, said particles are essentially spherical. In yet another or additional embodiment of the invention the superabsorbent polymer material has a relatively narrow range of particle sizes, e.g. with the majority (e.g. at least 80% or preferably at least 90% or even at least 95% by weight) of particles having a particle size between 50$\mu$m and 1000$\mu$m, preferably between 100$\mu$m and 800$\mu$m, and more preferably between 200$\mu$m and 600$\mu$m.

Suitable SAP may for example be obtained from inverse phase suspension polymerizations as described in US 4,340,706 and US 5,849,816 or from spray- or other gas-phase dispersion polymerizations as described in US Patent Applications No. 2009/0192035, 2009/0258994 and 2010/0068520. In some embodiments, suitable SAP may be obtained by current state of the art production processes as is more particularly described from page 12, line 23 to page 20, line 27 of WO 2006/083584.

The surface of the SAP may be coated, for example, with a cationic polymer. Preferred cationic polymers can include polyamine or polyimine materials. In some embodiments, the SAP may be coated with chitosan materials such as those disclosed in US 7,537,832 B2. In some other embodiments, the SAP may comprise mixed-bed Ion-Exchange absorbent polymers such as those disclosed in WO 99/34841 and WO 99/34842.

The absorbent core will typically comprise only one type of SAP, but it is not excluded that a blend of SAPs may be used. The fluid permeability of a superabsorbent polymer can be quantified using its Urine Permeability Measurement (UPM) value, as measured in the test disclosed European patent application number EP12174117.7. The UPM of the SAP may for example be of at least 10 x$10^{-7}$ cm$^3$.sec/g, or at least 30 x$10^{-7}$ cm$^3$.sec/g, or at least 50 x$10^{-7}$ cm$^3$.sec/g, or more, e.g. at least 80 or 100 x$10^{-7}$ cm$^3$.sec/g. The flow characteristics can also be adjusted by varying the quantity and distribution of the SAP used in the second absorbent layer.

For most absorbent articles, the liquid discharge occurs predominately in the front half of the article, in particular for diapers. The front half of the article (as defined by the region between the front edge and a transversal line placed at a distance of half L from the front or back edge) may therefore comprise most of the absorbent capacity of the core. Thus, at least 60% of the SAP, or at least 65%, or 70% of the SAP may be present in the front half of the absorbent article, the remaining SAP being disposed in the back half of the absorbent article. For other absorbent articles, the liquid discharge or more typically the liquid storage occurs predominately in the back half of the article, in particular for diapers for bedridden users. The back half of the article (as defined by the region between the back edge and a transversal line placed at a distance of half L from the front or back edge) may therefore comprise most of the absorbent capacity of the core. Thus, at least 60% of the SAP, or at least 65%, or 70% of the SAP may be present in the back half of the absorbent article, the remaining SAP being disposed in the front half of the absorbent article.

The total amount of SAP present in the absorbent core may also vary according to expected usage. Diapers for newborns may require less SAP than infant or adult incontinence diapers. The amount of SAP in the core may be for example comprised from about 2 to 60 g, in particular from 5 to 50 g or 10 to 40 g. It is useful that at least 60% or 70% or 80% or 90% or 95% of the SAP comprised by the core is present in the second (lower) core layer. The average SAP basis weight within the (or "at least one", if several are present) deposition area of the SAP may be for example of at least 50, 100, 200, 300, 400, 500 or more g/m$^2$. The areas of the channels present in the absorbent material deposition area, if any, are deduced from the absorbent material deposition area to calculate this average basis weight.

**Core wrap 66**

The optional core wrap may be made of a single substrate folded around the absorbent material, or may advantageously comprise two (or more) substrates which are attached to another. Typical attachments are the so-called C-wrap and/or sandwich wrap. In a C-wrap, as exemplarily shown in Fig. 2, the longitudinal and/or transversal edges of one of the substrate are folded over the other substrate to form flaps. These flaps are then bonded to the external surface of the other substrate, typically by gluing.

The optional core wrap may be formed by any materials suitable for receiving and containing the absorbent material. Typical substrate materials used in the production of conventional cores may be used, in particular paper, tissues, films, wovens or nonwovens, or laminate of any of these. The core wrap may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US 7,744,576, US 2011/0268932A1, US 2011/0319848A1 or US 2011/0250413A1. Nonwoven materials provided from synthetic fibers may be used, such as PE, PET and in particular PP.

If the core wrap comprises a first substrate 66a and a second substrate 66b these may be made of the same type of material, or may be made of different materials or one of the substrate may be treated differently than the other to provide it with different properties. As the polymers used for nonwoven production are inherently hydrophobic, they are preferably coated with hydrophilic coatings if placed on the fluid receiving side of the absorbent core. It is advantageous that the top side of the core wrap, i.e. the side placed closer to the wearer in the absorbent article, be more hydrophilic than the bottom side of the core wrap. A possible way to produce nonwovens with durably hydrophilic coatings is via applying a hydrophilic monomer and a radical polymerization initiator onto the nonwoven, and conducting a polymerization activated via UV light resulting in monomer chemically bound to the surface of the nonwoven. An alternative possible way to produce nonwovens with durably hydrophilic coatings is to coat the nonwoven with hydrophilic nanoparticles, e.g. as described in WO 02/064877.

Permanently hydrophilic nonwovens are also useful in some embodiments. Surface tension, as described in US 7744576 (Busam et al.), can be used to measure how permanently a certain hydrophilicity level is achieved. Liquid strike through, as described in US 7744576, can be used to measure the hydrophilicity level. The first and/or second substrate may in particular have a surface tension of at least 55, preferably at least 60 and most preferably at least 65 mN/m or higher when being wetted with saline solution. The substrate may also have a liquid strike through time of less than 5 s for a fifth gush of liquid. These values can be measured using the test methods described in US 7,744,576B2: "Determination Of Surface Tension" and "Determination of Strike Through" respectively.

Hydrophilicity and wettability are typically defined in terms of contact angle and the strike through time of the fluids, for example through a nonwoven fabric. This is discussed in detail in the American Chemical Society publication entitled "Contact angle, wettability and adhesion", edited by Robert F. Gould (Copyright 1964). A substrate having a lower contact angle between the water and the surface of substrate may be said to be more hydrophilic than another.

The substrates may also be air-permeable. Films useful herein may therefore comprise micro-pores. The substrate may have for example an air-permeability of from 40 or from 50, to 300 or to 200 m$^3$/ (m$^2$x min), as determined by EDANA method 140-1-99 (125 Pa, 38.3 cm$^2$). The material of the core wrap may alternatively have a lower air-permeability, e.g. being non-air-permeable, for example to facilitate handling on a moving surface comprising vacuum.

The core wrap may be at least partially sealed along all the sides of the absorbent core so that substantially no absorbent material leaks out of the core while performing the RCWR Test indicated below. By "substantially no absorbent material" it is meant that less than 5%, advantageously less than 2%, or less than 1% or 0% by weight of absorbent material escape the core wrap. In particular the core wrap should not in an appreciate way burst open while the test is conducted. The term "seal" is to be understood in a broad sense. The seal does not need to be continuous along the whole periphery of the core wrap but may be discontinuous along part or the whole of it, such as formed by a series of seal points spaced on a line. Typically a seal may be formed by gluing and/or thermal bonding.

If the core wrap is formed by two substrates 66a, 66b, four seals may be typically be used to enclose the absorbent material within the core wrap. For example, a first substrate 66a may be placed on one side of the core (the top side as represented in the Figures) and extends around the core's longitudinal edges to at least partially wrap the opposed bottom side of the core. The second substrate 66b is typically present between the wrapped flaps of the first substrate 66a and the absorbent material. The flaps of the first substrate 66a may be glued to the second substrate 66b to provide a strong seal. This so called C-wrap construction can provide benefits such as improved resistance to bursting in a wet loaded state compared to a sandwich seal. The front side and back side of the core wrap may then also be sealed for example by gluing the first substrate and second substrate to another to provide complete encapsulation of the absorbent material across the whole of the periphery of the core. For the front side and back side of the core the first and second substrate may extend and be joined together in a substantially planar direction, forming for these edges a so-called sandwich construction. In the so-called sandwich construction, the first and second substrates may also extend outwardly on all sides of the core and be sealed flat along the whole or parts of the periphery of the core typically by gluing and/or heat/pressure bonding. Typically neither first nor second substrates need to be shaped, so that they can be rectangularly cut for ease of production but of course other shapes are possible.

The core wrap may also be formed by a single substrate which may enclose as in a parcel wrap the absorbent material and be for example sealed along the front side and

**Barrier leg cuffs 30**

The absorbent article comprises a pair of barrier leg cuffs 30. The barrier leg cuffs can be formed from a piece of material, typically a nonwoven, which is partially bonded to the rest of the article so that a portion of the material, the barrier leg cuffs, can be partially raised away and stand up from the plane defined by the topsheet when the article is pulled flat as shown e.g. in Fig. 1. The barrier leg cuffs can provide improved containment of liquids and other body exudates approximately at the junction of the torso and legs of the wearer. The barrier leg cuffs extend at least partially between the front edge and the back edge of the diaper on opposite sides of the longitudinal axis and are at least present at the level of the crotch point (P). The barrier leg cuffs are delimited by a proximal edge 32 joined to the rest of the article, typically the topsheet and/or the backsheet, and a free terminal edge 66, which is intended to contact and form a seal with the wearer's skin. The barrier leg cuffs are joined at the proximal edge 32 with the chassis of the article by a bond 68 which may be made for example by gluing, fusion bonding or combination of known bonding means. The bond 68 at the proximal edge 32 may be continuous or intermittent. The side of the bond 68 closest to the raised section of the leg cuffs delimits the proximal edge 32 of the standing up section of the leg cuffs. The distance between the inner sides of these bond 68 define the dry and wet width of the article at this level for the purpose of RCWR test (see below).

The barrier leg cuffs can be integral with the topsheet or the backsheet, or more typically be formed from a separate material joined to the rest of the article. Typically the material of the barrier leg cuffs may extend through the whole length of the diapers but is "tack bonded" to the topsheet towards the front edge and back edge of the article so that in these sections the barrier leg cuff material remains flush with the topsheet. Each barrier leg cuff 30 may comprise one, two or more elastic 36 close to this free distal edge 34 to provide a better seal.

In addition to the barrier leg cuffs 30, the article may comprise gasketing cuffs 26, which are joined to the chassis of absorbent article, in particular the topsheet and/or the backsheet and are placed transversely outwardly relative to the barrier leg cuffs. The gasketing cuffs can provide a better seal around the thighs of the wearer. Usually each gasketing leg cuff will comprise one or more elastic string or elastic element comprised in the chassis of the diaper for example between the topsheet and backsheet in the area of the leg openings.

US 3,860,003 describes a disposable diaper which provides a contractible leg opening having a side flap and one or more elastic members to provide an elasticized leg cuff (a gasketing cuff). US 4,808,178 and US 4,909,803 issued to Aziz et al. describe disposable diapers having "stand-up" elasticized flaps (barrier leg cuffs) which improve the containment of the leg regions. US 4,695,278 and US 4,795,454 issued to Lawson and to Dragoo respectively, describe disposable diapers having dual cuffs, including gasketing cuffs and barrier leg cuffs. All or a portion of the barrier leg and/or gasketing cuffs may be treated with a lotion.

**Acquisition-distribution system 50**

The absorbent articles of the invention may comprise an acquisition-distribution layer or system 50 (herein "ADS"). The function of the ADS is to quickly acquire the fluid and distribute it to the absorbent core in an efficient manner.

The ADS may comprise one, two or more layers, which may form a unitary layer or remain discrete layers which may be attached to each other. In the examples below, the ADS comprises two layers: a distribution layer 54 and an acquisition layer 52 disposed between the absorbent core and the topsheet, but the invention is not restricted to this example.

Typically, the ADS will not comprise SAP as this may slow the acquisition and distribution of the fluid. The prior art discloses many type of acquisition-distribution system, see for example WO2000/59430 (Daley), WO95/10996 (Richards), US 5,700,254 (McDowall), WO02/067809 (Graef). The ADS may comprise, although not necessarily, two layers: a distribution layer and an acquisition layer, which will now be exemplified in more details.

Layers of the acquisition distribution system will typically be very permeable for liquid and therefore have high IPRP values. Such values are generally above 1200, useful layers have been found to have values above 1600, 2400 or sometimes also above 6000.

**Distribution layer 54**

The distribution layer may for example comprise at least 50% by weight of cross-linked cellulose fibers. The cross-linked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf. The cross-linked cellulosic fibers provide higher resilience and therefore higher resistance to the first absorbent layer against the compression in the product packaging or in use conditions, e.g. under baby weight. This provides the core with a higher void volume, permeability and liquid absorption, and hence reduced leakage and improved dryness.

Exemplary chemically cross-linked cellulosic fibers suitable for a distribution layer are disclosed in US 5,549,791, US 5,137,537, WO9534329 or US 2007/118087. Exemplary cross-linking agents include polycarboxylic acids such as citric acid and/or polyacrylic acids such as acrylic acid and maleic acid copolymers. For example, the crosslinked cellulosic fibers may have between about 0.5 mole % and about 10.0 mole % of a C2 -C9 polycarboxylic acid cross-linking agent, calculated on a cellulose anhydroglucose molar basis, reacted with said fibers in an intrafiber ester crosslink bond form. The C2 -C9 polycarboxylic acid cross-linking agent may be selected from the group consisting of:

- aliphatic and alicyclic C2 -C9 polycarboxylic acids having at least three carboxyl groups per molecule; and

- aliphatic and alicyclic C2 -C9 polycarboxylic acids having two carboxyl groups per molecule and having a carbon-carbon double bond located alpha, beta to one or both of the carboxyl groups, wherein one carboxyl group in said C2 -C9 polycarboxylic acid crosslinking agent is separated from a second carboxyl group by either two or three carbon atoms. The fibers may have in particular between about 1.5 mole % and about 6.0 mole % crosslinking agent, calculated on a cellulose anhydroglucose molar basis, reacted therewith in the form of intrafiber ester crosslink bonds. The cross-linking agent may be selected from the group consisting of citric acid, 1, 2, 3, 4 butane tetracarboxylic acid, and 1, 2, 3 propane tricarboxylic acid, in particular citric acid.

Polyacrylic acid cross-linking agents may also be selected from polyacrylic acid homopolymers, copolymers of acrylic acid, and mixtures thereof. The fibers may have between 1.0 weight % and 10.0 weight %, preferably between 3 weight % and 7 weight %, of these cross-linking agents, calculated on a dry fiber weight basis, reacted therewith in the form of intra-fiber crosslink bonds. The cross-linking agent may be a polyacrylic acid polymer having a molecular weight of from 500 to 40,000, preferably from 1,000 to 20,000. The polymeric polyacrylic acid cross-linking agent may be a copolymer of acrylic acid and maleic acid, in particular wherein the weight ratio of acrylic acid to maleic acid is from 10:1 to 1:1, preferably from 5:1 to 1.5:1. An effective amount of citric acid may be further mixed with said polymeric polyacrylic acid cross-linking agent.

The distribution layer comprising cross-linked cellulose fibers of the invention may comprise other fibers, but this layer may advantageously comprise at least 50%, or 60%, or 70%, or 80%, or 90% or even up to 100%, by weight of the layer, of cross-linked cellulose fibers (including the cross-linking agents). Examples of such mixed layer of cross-linked cellulose fibers may comprise about 70% by weight of chemically cross-linked cellulose fibers, about 10 % by weight polyester (PET) fibers, and about 20 % by weight untreated pulp fibers. In another example, the layer of cross-linked cellulose fibers may comprise about 70 % by weight chemically cross-linked cellulose fibers, about 20 % by weight lyocell fibers, and about 10% by weight PET fibers. In another example, the layer may comprise about 68 % by weight chemically cross-linked cellulose fibers, about 16 % by weight untreated pulp fibers, and about 16 % by weight PET fibers. In another example, the layer of cross-linked cellulose fibers may comprise from about 90-100% by weight chemically cross-linked cellulose fibers.

The distribution layer 54 may be a material having a water retention value of from 25 to 60, preferably from 30 to 45, measured as indicated in the procedure disclosed in US 5,137,537.

The distribution layer may typically have an average basis weight of from 30 to 400 g/m$^2$, in particular from 100

to 300 g/m$^2$. The density of the distribution layer may vary depending on the compression of the article, but may be of between 0.03 to 0.15 g/cm$^3$, in particular 0.08 to 0.10 g/cm$^3$ measured at 0.30 psi (2.07kPa).

A useful distribution layer has been found to have IPRP values in the range of 1200 to 5000, often 1600 to 4500, or 2400 to 4000.

**Acquisition layer 52**

The ADS may comprise an acquisition layer 52. The acquisition layer may be disposed between the distribution layer 54 and topsheet 22. The acquisition layer 52 may typically be or comprise a non-woven material, for example a SMS or SMMS material, comprising a spunbonded, a melt-blown and a further spunbonded layer or alternatively a carded chemical-bonded nonwoven. The non-woven material may in particular be latex bonded. Exemplary upper acquisition layers 52 are disclosed in US 7,786,341. Carded, resin-bonded nonwovens may be used, in particular where the fibers used are solid round or round hollow PET staple fibers (50/50 or 40/60 mix of 6 denier and 9 denier fibers). An exemplary binder is a butadiene/styrene latex. Non-wovens have the advantage that they can be manufactured outside the converting line and stored and used as a roll of material.

Further useful non-wovens are described in U.S. Pat. No. 6,645,569 to Cramer et al., U.S. Patent No. 6,863,933 to Cramer et al., U.S. Patent No. 7,112,621 to Rohrbaugh et al., and co patent applications US 2003/148684 to Cramer et al. and US 2005/008839 to Cramer et al.

The acquisition layer 52 may be stabilized by a latex binder, for example a styrene-butadiene latex binder (SB latex). Processes for obtaining such lattices are known, for example, from EP 149 880 (Kwok) and US 2003/0105190 (Diehl et al.). In certain embodiments, the binder may be present in the acquisition layer 52 in excess of about 12%, about 14% or about 16% by weight. SB latex is available under the trade name GENFLO™ 3160 (OMNOVA Solutions Inc.; Akron, Ohio).

A further acquisition layer may be used in addition to a first acquisition layer described above. For example a tissue layer may be placed between the first acquisition layer and the distribution layer. The tissue may have enhanced capillarity distribution properties compared to the acquisition layer described above. The tissue and the first acquisition layer may be of the same size or may be of different size, for example the tissue layer may extend further in the back of the absorbent article than the first acquisition layer. An example of hydrophilic tissue is a 13 - 15 gsm high wet strength made of cellulose fibers from supplier Havix.

Useful acquisition layers can also be provided as formed film patches.

A useful acquisition layer has been found to have IPRP values in the range of 5000 to 30000, often 10000 to 25000.

**Fastening system**

The absorbent article may include a fastening system. The fastening system can be used to provide lateral tensions about the circumference of the absorbent article to hold the absorbent article on the wearer as is typical for taped diapers. This fastening system is not necessary for training pant article since the waist region of these articles is already bonded. The fastening system usually comprises a fastener such as tape tabs (also referred to as adhesive tabs), hook and loop fastening components, interlocking fasteners such as tabs & slots, buckles, buttons, snaps, and/or hermaphroditic fastening components, although any other known fastening means are generally acceptable. A landing zone is normally provided on the front waist region for the fastener to be releasably attached. Some exemplary surface fastening systems are disclosed in US 3,848,594, US 4,662,875, US 4,846,815, US 4,894,060, US 4,946,527, US 5,151,092 and US 5,221,274 issued to Buell. An exemplary interlocking fastening system is disclosed in US 6,432,098. The fastening system may also provide a means for holding the article in a disposal configuration as disclosed in US 4,963,140 issued to Robertson et al.

The fastening system may also include primary and secondary fastening systems, as disclosed in US 4,699,622 to reduce shifting of overlapped portions or to improve fit as disclosed in US 5,242,436, US 5,499,978, US 5,507,736, and US 5, 591,152.

**Back ears 40 and front ears 46**

The absorbent article may comprise front ears 46 and back ears 40 as is known in the art. The ears can be integral part of the chassis, for example formed from the topsheet and/or backsheet as side panel. Alternatively, as represented on Fig. 1, they may be separate elements attached by gluing and / or heat embossing or pressure bonding. The back ears 40 are advantageously stretchable to facilitate the attachment of the adhesive tabs 42 on the landing zone 44 and maintain the taped diapers in place around the wearer's waist. The back ears 40 may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

**Elastic waist feature**

The absorbent article may also comprise at least one elastic waist feature (not represented) that helps to provide improved fit and containment. The elastic waist feature is generally intended to elastically expand and contract to dynamically fit the wearer's waist. The elastic waist feature preferably extends at least longitudinally outwardly from at least one waist edge of the absorbent core 20 and generally forms at least a portion of the end edge of the absorbent article. Disposable diapers can be constructed so as to have two elastic waist features, one positioned in the front waist region and one positioned in the back waist region. The elastic waist feature may be constructed in a number of different configurations including those described in US 4,515,595, US 4,710,189, US 5,151,092 and US 5,221,274.

**Relations between the layers**

Typically, adjacent layers and components will be joined together using conventional bonding method such as adhesive coating via slot coating or spraying on the whole or part of the surface of the layer, or thermo-bonding, or pressure bonding or combinations thereof. This bonding is not represented in the Figures (except for the bonding by bonds 68 between the raised elements of the barrier leg cuffs 30 with the topsheet 22) for clarity and readability but bonding between the layers of the article should be considered to be present unless specifically excluded. Adhesives may be typically used to improve the adhesion of the different layers, for example between the backsheet and the core wrap. The glue may be any standard hotmelt glue as known in the art.

If an acquisition layer 52 is present, it may be advantageous that this acquisition layer is larger than or least as large as the distribution layer 54 in the longitudinal and/or transversal dimension. In this way the distribution layer 54 can be deposited on the acquisition layer 52 during the manufacturing process before assembling these layers in the finished article. This simplifies handling, in particular if the acquisition layer is a nonwoven which can be unrolled from a roll of stock material. The distribution layer may also be deposited directly on the absorbent core's upper side of the core wrap or another layer of the article. Also, an acquisition layer 52 larger than the distribution layer allows to directly glue the acquisition layer to the storage core (at the larger areas). This can give increased integrity to the article and better liquid communication.

The absorbent core and in particular its absorbent material deposition area may advantageously be at least as large and long and advantageously at least partially larger and/or longer than the acquisition-distribution system (ADS). This is because the absorbent material in the core can usually more effectively retain fluid and provide dryness benefits across a larger area than the ADS. The absorbent article may have a rectangular SAP layer and a non-rectangular (shaped) ADS. The absorbent article may also have a rectangular (non-shaped) ADS and a rectangular layer of SAP.

**Method of making the article**

The absorbent articles of the invention may be made by any conventional methods known in the art. In particular the articles may be hand-made or industrially produced at high speed.

**Experimental settings**

The values indicated herein are measured according to the methods indicated herein below, unless specified otherwise. All measurements are performed at 21 $\pm$ 2°C and 50 $\pm$ 20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

CALIPER TEST

Equipment: Mitutoyo manual caliper gauge with a resolution of 0.01 mm -- or equivalent instrument.

Contact Foot: Flat circular foot with a diameter of 17.0 mm ($\pm$ 0.2 mm). A circular weight may be applied to the foot (e.g., a weight with a slot to facilitate application around the instrument shaft) to achieve the target weight. The total weight of foot and added weight (including shaft) is selected to provide 2.07 kPa (0.30 psi) of pressure to the sample.

If there was a spring present to push the foot to the sample the spring is removed from the equipment, such that indeed the equipment applies a pressure of 2.07 kPa.

The caliper gauge is mounted with the lower surface of the contact foot in an horizontal plane so that the lower surface of the contact foot contacts the center of the flat horizontal upper surface of a base plate approximately 20 x 25 cm. The gauge is set to read zero with the contact foot resting on the base plate.

Ruler: Calibrated metal ruler graduated in mm.

Stopwatch: Accuracy 1 second

Sample preparation:

If the absorbent articles are provided in a package, the sample articles to be tested are removed from the center area of a package. If the package contains more than 4 articles, the outer most two articles on each side of the package are not used in the testing. If the package contains more than 4 but fewer than 14 articles, then more than one package of articles is required to complete the testing. If the package contains 14 or more articles, then only one package of articles is required to perform the testing. If the package contains 4 or fewer articles then all articles in the package are measured and multiple packages are required to perform the measurement. Caliper readings should be taken $24 \pm 1$ hours after the article is removed from the package. Physical manipulation of product should be minimal and restricted only to necessary sample preparation.
Any elastic components of the article that prevent the article from being laid flat under the caliper foot are cut or removed. These may include leg cuffs or waistbands. Pant-type articles are opened or cut along the side seams as necessary. Apply sufficient tension to flatten out any folds/wrinkles. Care is taken to avoid touching and/or compressing the absorbent core and ADS area.

Measurement procedure:

The article is laid flat on a counter top, garment-facing side down. A lateral line is drawn across the body-facing surface of the article at the level of the crotch point P.
The contact foot of the caliper gauge is raised and the article is placed on base plate, garment-facing surface side down so that when lowered, the center of the foot is on marked measuring point at the crotch point P.
The foot is gently lowered onto the article and released (ensure calibration to "0" prior to the start of the measurement). The caliper value is read to the nearest 0.01 mm, 10 seconds after the foot is released.
The procedure is repeated for each measuring point. If there is a fold at the measuring point, the measurement is done in the closest area to this point but without any folds. Ten articles are measured in this manner for a given product and the average caliper is calculated and reported with an accuracy of one tenth mm.

IPRP ANALYSIS

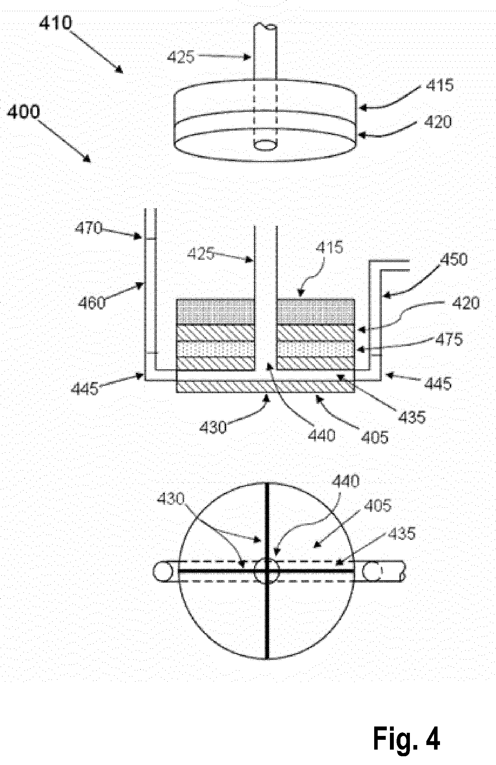

Permeability generally refers to the quality of a porous material that causes it to allow liquids or gases to pass through it and, as such, is generally determined from the mass flow rate of a given fluid through it. The permeability of an absorbent structure is related to the material's ability to quickly acquire and transport a liquid within the structure, both of which are key features of an absorbent article. Accordingly, measuring permeability is one metric by which a material's suitability for use in absorbent articles may be assessed.
The following test is suitable for measurement of the In-Plane Radial Permeability (IPRP) of a porous material. The quantity of a saline solution (0.9% NaCl) flowing radially through an annular sample of the material under constant pressure is measured as a function of time.
Testing is performed at $23°C \pm 2C°$ and a relative humidity $50\% \pm 5\%$. All samples are conditioned in this environment for twenty four (24) hours before testing.
The IPRP sample holder 400 is shown in FIG. 4 and comprises a cylindrical bottom plate 405, top plate 410, and cylindrical stainless steel weight 415.
Top plate 410 comprises an annular base plate 420, which is 9 mm thick with an outer diameter of 70 mm and a tube 425 of 150 mm length fixed at the center thereof. The tube 425 has an outer diameter of 15.8 mm and an inner diameter of 12 mm. The tube is adhesively fixed into a circular 16 mm hole in the center of the base plate 420 such that the lower edge of the tube is flush with the lower surface of the base plate, as depicted in FIG. 4. The bottom plate 405 and top plate 410 are fabricated from Lexan® or equivalent. The stainless steel weight 415 has an outer diameter of 70 mm and an inner diameter of 15.9 mm so that the weight is a close sliding fit on tube 425. The thickness of the stainless steel weight 415 is approximately 22 mm and is adjusted so that the total weight of the top plate 410 and the stainless steel weight 415 is 687g $\pm$ 1 g to provide 2.0 kPa of confining pressure during the measurement.

Bottom plate 405 is approximately 25 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 405 has a horizontal hole 435 which spans the diameter of the plate. The horizontal hole 435 has a diameter of 8 mm and its central axis is 15 mm below the upper surface of bottom plate 405. Bottom plate 405 also has a central vertical hole 440 which has a diameter of 8 mm and is 10 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a total height of 175 mm measured from the bottom of bottom plate 405 (including elbow 445) and an internal diameter of 6 mm. The tube 460 is scribed with a suitable mark 470 at a height of 100 mm above the upper surface of the bottom plate 420. This is the reference for the fluid level to be maintained during the measurement. The other elbow 445 is connected to the fluid delivery reservoir 700 (described below) via a flexible tube.

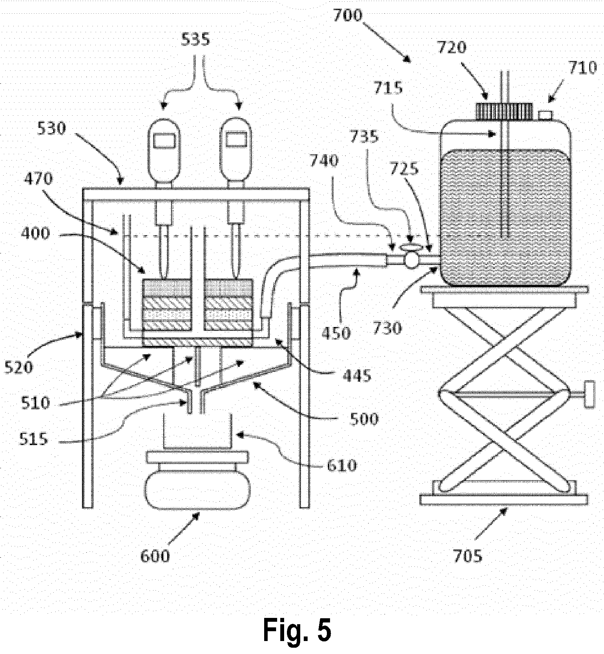

A suitable fluid delivery reservoir 700 is shown in FIG. 5. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 740. The outlet 740 is connected to elbow 445 via flexible plastic tubing 450 (e.g. Tygon®). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 liters, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.

The IPRP catchment funnel 500 is shown in FIG. 5 and comprises an outer housing 505 with an internal diameter at the upper edge of the funnel of approximately 125 mm. Funnel 500 is constructed such that liquid falling into the funnel drains rapidly and freely from spout 515. A stand with horizontal flange 520 around the funnel 500 facilitates mounting the funnel in a horizontal position. Two integral vertical internal ribs 510 span the internal diameter of the funnel and are perpendicular to each other. Each rib 510 is 1.5 mm wide and the top surfaces of the ribs lie in a horizontal plane. The funnel housing 500 and ribs 510 are fabricated from a suitably rigid material such as Lexan® or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount two digital calipers 535 to measure the relative height of the stainless steel weight 415. The digital calipers 535 have a resolution of $\pm$ 0.01 mm over a range of 25 mm. A suitable digital caliper is a Mitutoyo model 543-492B or equivalent. Each caliper is interfaced with a computer to allow height readings to be recorded periodically and stored electronically on the computer. Bridge 530 has a circular hole 22 mm in diameter to accommodate tube 425 without the tube touching the bridge.

Funnel 500 is mounted over an electronic balance 600, as shown in FIG. 5. The balance has a resolution of $\pm$ 0.01 g and a capacity of at least 1000 g. The balance 600 is also interfaced with a computer to allow the balance reading to be recorded periodically and stored electronically on the computer. A suitable balance is Mettler-Toledo model MS6002S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel spout 515 falls directly into the container 610. The IPRP sample holder 400 is situated centrally in the funnel 500 with the ribs 510 located in grooves 430. The upper surface of the bottom plate 405 must be perfectly flat and level. The top plate 410 is aligned with and rests on the bottom plate 405. The stainless steel weight 415 surrounds the tube 425 and rests on the top plate 410. Tube 425 extends vertically through the central hole in the bridge 530. Both calipers 535 are mounted firmly to the bridge 530 with the foot resting on a point on the upper surface of the stainless steel weight 415. The calipers are set to zero in this state. The reservoir 700 is filled with 0.9% saline solution and re-sealed. The outlet 740 is connected to elbow 445 via flexible plastic tubing 450.

An annular sample 475 of the material to be tested is cut by suitable means. The sample has an outer diameter of 70 mm and an inner hole diameter of 12 mm. One suitable means of cutting the sample is to use a die cutter with sharp concentric blades.

The top plate 410 is lifted enough to insert the sample 475 between the top plate and the bottom plate 405 with the sample centered on the bottom plate and the plates aligned. The stopcock 735 is opened and the level of fluid in tube 460 is set to the scribed mark 470 by adjusting the height of the reservoir 700 using the jack 705 and by adjusting the position of the tube 715 in the reservoir. When the fluid level in the tube 460 is stable at the scribed mark 470 initiate recording data from the balance and calipers by the computer. Balance readings and time elapsed are recorded every 10 seconds for five minutes. The average sample thickness B is calculated from all caliper reading between 60 seconds and 300 seconds and expressed in cm. The flow rate in grams per second is the slope calculated by linear least squares regression fit of the balance reading (dependent variable) at different times (independent variable) considering only the readings between 60 seconds and 300 seconds.

Permeability $k$ is then calculated by the following equation:

$$k = \frac{(Q/\rho_l)\cdot \mu \cdot \ln(R_0/R_i)}{2\pi \cdot B \cdot \Delta p} \tag{1}$$

Where:

$k$ is the permeability ($cm^2$);
$Q$ is the flow rate (g/s);
$\rho_1$ is the liquid density at 20°C ($g/cm^3$);
$\mu$ is the liquid viscosity at 20 °C (Pa·s);
Ro is the outer sample radius (cm);
$R_i$ is the inner sample radius (cm);
$B$ is the average sample thickness (cm); and
$\Delta_p$ is the pressure drop (Pa) calculated according to the following equation:

$$\Delta p = \left(\Delta h - \frac{B}{2}\right)\cdot g \cdot \rho_l \cdot 10 \tag{2}$$

Where:

$\Delta h$ is the measured liquid hydrostatic pressure (cm);
$g$ is the acceleration constant ($m/sec^2$); and
$\rho_1$ is the liquid density ($g/cm^3$).

IPRP is dependent on the fluid being used, so the IPRP value (in $cm^2$/MPa·sec) may be defined and calculated as follows:

$$IPRP\ value = (k/\mu) \tag{3}$$

Where:

k is the permeability ($cm^2$),
$\mu$ is the liquid viscosity at 20 °C (MPa·s). ***

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. An absorbent article such as a diaper (10), or training pant, or incontinence insert, the absorbent article having a front edge (12) and a rear edge (14), a longitudinal axis extending in a longitudinal direction of the article, the article having a length L as measured along the longitudinal axis from the front edge to the back edge, the absorbent article comprising:

   - an essentially liquid permeable topsheet (22),
   - an essentially liquid impermeable backsheet (24),
   - an absorbent core (20) between the topsheet (22) and backsheet (24),
   - an acquisition-distribution system (50) which may comprise one or several layers (52, 54) and which is at least partially disposed between the absorbent core (20) and the topsheet (22),
   - wherein the an absorbent core (20) comprises a first core layer (56) and a second core layer (58),
   - the first core layer (56) being arranged closer to the topsheet (22) than the second core layer (58),
   - the second core layer (58) extends over a length L2 from a front edge to a rear edge and over a first segment, a second segment, a third segment, a fourth segment, a fifth segment, and a sixth segment, the first segment, the second segment, the third segment, the fourth segment, the fifth segment, and the sixth segment all being of equal length, the a first segment comprising the front edge of the second core layer (58) and the sixth segment comprising the rear edge of the second core layer (58),
   - the first core layer (56) comprises an orifice (70),
   - the second core layer (58) comprises a superabsorbent material (62),
   - the second core layer (58) comprises a first amount of superabsorbent material (62) in the first segment, a second amount of superabsorbent material (62) in the second segment, a third amount of superabsorbent material (62) in the third segment, a fourth amount of superabsorbent material (62) in the fourth segment, a fifth amount of superabsorbent material (62) in the fifth segment and a sixth amount of superabsorbent material (62) in the sixth segment,
   - the fourth amount is from 0% to 80% of the fifth amount.

2. An absorbent article according to the preceding claim wherein the third amount is from 0% to 80% of the second amount.

3. An absorbent article according to any one of the preceding claims wherein the fourth amount is from 40% to 70% of the fifth amount.

4. An absorbent article according to any one of the preceding claims wherein the third amount is from 40% to 70% of the second amount.

5. An absorbent article according to any one of the preceding claims wherein the first core layer (56) comprises a fibrous absorbent material (60).

6. An absorbent article according to any one of the preceding claims wherein the first core layer (56) comprises superabsorbent material (62).

7. An absorbent article according to the preceding claim wherein the superabsorbent material (62) is distributed evenly over the longitudinal direction of the core in the first core layer (56).

8. An absorbent article according to any one of the preceding claims wherein the first core layer (56) comprises an orifice (70).

9. An absorbent article according to the preceding claim wherein the orifice (70) comprises a minimal open length of 5 mm.

10. An absorbent article according to the preceding claim wherein the orifice (70) comprises a maximal open length of 200 mm.

11. An absorbent article according to the preceding claim wherein the orifice (70) is of oblong shape.

12. An absorbent article according to the preceding claim wherein the orifice (70) is positioned, fully or in part, above the third segment or above the fourth segment or above both the third segment and the fourth segment.

13. An absorbent article according to the preceding claim wherein the second core layer (58) comprises a fibrous absorbent material (64) comprising the superabsorbent material (62).

14. An absorbent article according to any one of the preceding claims wherein the second core layer (58) has an IPRP value of less than 1200.

15. An absorbent article according to any of the preceding claims wherein the absorbent article is a diaper or an incontinence insert.

**Fig. 1**

Fig. 2

**Fig. 3**

Fig. 4

Fig. 5

Europäisches
Patentamt

European
Patent Office

Office européen
des brevets

# EUROPEAN SEARCH REPORT

**Application Number**

EP 16 18 2344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | EP 0 439 012 A2 (KIMBERLY CLARK CO [US])<br>31 July 1991 (1991-07-31)<br>* column 1, lines 3-10 *<br>* column 3, lines 48-58 *<br>* column 22, lines 20-31 *<br>* column 26, lines 22-28 *<br>* column 27, lines 5-8 *<br>* column 28, line 14 - column 29, line 2 *<br>* column 29, line 50 - column 30, line 7 *<br>* column 32, lines 43-52; figure 16 *<br>----- | 1,2,8<br><br>3-7,9-15 | INV.<br>A61F13/532<br>A61F13/535<br>A61F13/53 |
| Y | WO 2013/185800 A1 (SCA HYGIENE PROD AB [SE]; FREDRIKSON SUSANNE [SE]; JOHANSSON CHARLOTTE) 19 December 2013 (2013-12-19)<br>* figure 5 *<br>----- | 1,2,8 | |
| A | EP 2 886 094 A1 (PROCTER & GAMBLE [US])<br>24 June 2015 (2015-06-24)<br>* page 2, paragraph 6 - page 3, paragraph 8 *<br>* page 3, paragraph 11 *<br>* page 5, paragraph 24 *<br>* page 6, paragraph 29 - page 7, paragraph 34 *<br>* page 9, paragraphs 46,50 *<br>* page 12, paragraph 69 *<br>* figures 1,2,8,9 *<br>----- | 1-15 | |
| A | EP 2 671 554 A1 (PROCTER & GAMBLE [US])<br>11 December 2013 (2013-12-11)<br>* page 8, paragraphs 48,49 *<br>----- | 1-15 | |
| A | EP 0 325 416 A2 (PROCTER & GAMBLE [US])<br>26 July 1989 (1989-07-26)<br>* page 2, lines 8-12 *<br>* page 3, lines 1-31 *<br>* page 12, lines 3-22 *<br>----- | 1-15 | |

**TECHNICAL FIELDS SEARCHED (IPC)**

A61F

-/--

The present search report has been drawn up for all claims

| Place of search | Date of completion of the search | Examiner |
|---|---|---|
| Munich | 27 January 2017 | Beins, Ulrika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 18 2344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 873 867 A (COLES PETER [DE] ET AL) 23 February 1999 (1999-02-23) * column 1, lines 5-18 * * column 5, lines 30-45 * * column 6, lines 12-17 * * column 8, line 55 - column 9, line 7 * * column 10, lines 11-22 * * column 13, lines 14-27 * ----- | 1-15 | |
| A | EP 2 740 454 A1 (PROCTER & GAMBLE [US]) 11 June 2014 (2014-06-11) * figures 2,3 * ----- | 1-15 | |

TECHNICAL FIELDS
SEARCHED       (IPC)

The present search report has been drawn up for all claims

| Place of search | Date of completion of the search | Examiner |
|---|---|---|
| Munich | 27 January 2017 | Beins, Ulrika |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

............................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 18 2344

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2017

| Patent document cited in search report | | Publication date | Patent family member(s) | | Publication date |
|---|---|---|---|---|---|
| EP 0439012 | A2 | 31-07-1991 | AT | 128618 T | 15-10-1995 |
| | | | AT | 150637 T | 15-04-1997 |
| | | | AU | 634330 B2 | 18-02-1993 |
| | | | AU | 649365 B2 | 19-05-1994 |
| | | | AU | 667774 B2 | 04-04-1996 |
| | | | BR | 9100040 A | 22-10-1991 |
| | | | CA | 2016411 A1 | 09-07-1991 |
| | | | DE | 69113485 D1 | 09-11-1995 |
| | | | DE | 69113485 T2 | 28-03-1996 |
| | | | DE | 69125402 D1 | 30-04-1997 |
| | | | DE | 69125402 T2 | 10-07-1997 |
| | | | EP | 0439012 A2 | 31-07-1991 |
| | | | EP | 0642778 A1 | 15-03-1995 |
| | | | ES | 2080166 T3 | 01-02-1996 |
| | | | ES | 2100004 T3 | 01-06-1997 |
| | | | US | 5028224 A | 02-07-1991 |
| | | | ZA | 9100048 B | 30-10-1991 |
| WO 2013185800 | A1 | 19-12-2013 | AU | 2012382613 A1 | 15-01-2015 |
| | | | AU | 2013276741 A1 | 15-01-2015 |
| | | | CA | 2875400 A1 | 19-12-2013 |
| | | | CA | 2875443 A1 | 19-12-2013 |
| | | | CN | 104379105 A | 25-02-2015 |
| | | | CN | 104394819 A | 04-03-2015 |
| | | | EP | 2858615 A1 | 15-04-2015 |
| | | | ES | 2569115 T3 | 06-05-2016 |
| | | | JP | 2015519154 A | 09-07-2015 |
| | | | JP | 2015519157 A | 09-07-2015 |
| | | | KR | 20150020680 A | 26-02-2015 |
| | | | KR | 20150020681 A | 26-02-2015 |
| | | | PL | 2858616 T3 | 30-09-2016 |
| | | | RU | 2014153439 A | 27-07-2016 |
| | | | RU | 2014153526 A | 10-08-2016 |
| | | | TW | 201402087 A | 16-01-2014 |
| | | | US | 2015148769 A1 | 28-05-2015 |
| | | | US | 2015173971 A1 | 25-06-2015 |
| | | | WO | 2013185800 A1 | 19-12-2013 |
| | | | WO | 2013186098 A1 | 19-12-2013 |
| EP 2886094 | A1 | 24-06-2015 | CA | 2933842 A1 | 25-06-2015 |
| | | | CN | 105828774 A | 03-08-2016 |
| | | | EP | 2886094 A1 | 24-06-2015 |
| | | | US | 2015174280 A1 | 25-06-2015 |
| | | | WO | 2015095003 A1 | 25-06-2015 |
| EP 2671554 | A1 | 11-12-2013 | CN | 104470482 A | 25-03-2015 |

EPO FORM P0459

For more details about this annex : see Official Journal of the European Patent Office, No. 12/82

**ANNEX TO THE EUROPEAN SEARCH REPORT**
**ON EUROPEAN PATENT APPLICATION NO.**                    EP 16 18 2344

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2017

| Patent document cited in search report | | Publication date | Patent family member(s) | | Publication date |
|---|---|---|---|---|---|
| | | | EP | 2671554 A1 | 11-12-2013 |
| | | | JP | 6009660 B2 | 19-10-2016 |
| | | | JP | 2015518773 A | 06-07-2015 |
| | | | US | 2013331806 A1 | 12-12-2013 |
| | | | US | 2016296386 A1 | 13-10-2016 |
| | | | WO | 2013184859 A1 | 12-12-2013 |
| EP 0325416 | A2 | 26-07-1989 | AT | 105703 T | 15-06-1994 |
| | | | CA | 1314696 C | 23-03-1993 |
| | | | DE | 68915294 D1 | 23-06-1994 |
| | | | DE | 68915294 T2 | 27-10-1994 |
| | | | EP | 0325416 A2 | 26-07-1989 |
| | | | ES | 2052896 T3 | 16-07-1994 |
| | | | JP | H025945 A | 10-01-1990 |
| | | | JP | 2996664 B2 | 11-01-2000 |
| US 5873867 | A | 23-02-1999 | NONE | | |
| EP 2740454 | A1 | 11-06-2014 | CA | 2894680 A1 | 19-06-2014 |
| | | | CL | 2015001582 A1 | 02-10-2015 |
| | | | CN | 104853707 A | 19-08-2015 |
| | | | EP | 2740454 A1 | 11-06-2014 |
| | | | JP | 2016502871 A | 01-02-2016 |
| | | | US | 2014163504 A1 | 12-06-2014 |
| | | | WO | 2014093323 A1 | 19-06-2014 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151092 A, Buell
- WO 2008155699 A, Hundorf
- WO 9511652 A, Tanzer
- WO 2012052172 A, Van Malderen
- US 4808178 A
- US 4909803 A, Aziz
- US 4695278 A, Lawson
- US 4795454 A, Dragoo
- US 4704116 A, Enloe
- US 3860003 A
- US 5221274 A
- US 5554145 A
- US 5569234 A
- US 5580411 A
- US 6004306 A
- US 3929135 A
- US 4324246 A
- US 4342314 A
- US 4463045 A
- US 5006394 A
- US 4609518 A
- US 4629643 A, Curro
- US 5607760 A
- US 5609587 A
- US 5643588 A
- US 5968025 A
- US 6716441 B
- WO 9524173 A
- US 6632504 B
- WO 2011163582 A
- WO 9516746 A
- US 5938648 A, LaVon
- US 4681793 A
- US 5865823 A, Curro
- US 5571096 A, Dobrin
- US 6946585 B2, London Brown
- US 4573986 A
- US 3911173 A
- US 4785996 A
- US 4842666 A
- WO 20100051166 A1
- US 5599335 A, Goldman
- EP 1447066 A, Busam
- US 20080312622 A1, Hundorf
- US 4731066 A, Korpman
- WO 201027719 A
- WO 07047598 A
- WO 07046052 A
- WO 2009155265 A

- WO 2009155264 A
- WO 2006083584 A
- EP 530438 A
- EP 547847 A
- EP 559476 A
- EP 632068 A
- WO 9321237 A
- WO 03104299 A
- WO 03104300 A
- WO 03104301 A
- DE 10331450 A
- DE 10331456 A
- DE 10355401 A
- DE 19543368 A
- DE 19646484 A
- WO 9015830 A
- WO 0232962 A
- EP 083022 A
- EP 543303 A
- EP 937736 A
- DE 3314019 C
- DE 4020780 A
- DE 19807502 A
- DE 19807992 A
- DE 19854573 A
- DE 19854574 A
- DE 10204937 A
- DE 10334584 A
- EP 1199327 A
- WO 03031482 A
- EP 0691133 A
- US 4340706 A
- US 5849816 A
- US 20090192035 A
- US 20090258994 A
- US 20100068520 A
- US 7537832 B2
- WO 9934841 A
- WO 9934842 A
- EP 12174117 A
- US 7744576 B
- US 20110268932 A1
- US 20110319848 A1
- US 20110250413 A1
- WO 02064877 A
- US 7744576 B2
- WO 200059430 A, Daley
- WO 9510996 A, Richards
- US 5700254 A, McDowall

- WO 02067809 A, Graef
- US 5549791 A
- US 5137537 A
- WO 9534329 A
- US 2007118087 A
- US 7786341 B
- US 6645569 B, Cramer
- US 6863933 B, Cramer
- US 7112621 B, Rohrbaugh
- US 2003148684 A, Cramer
- US 2005008839 A, Cramer
- EP 149880 A, Kwok
- US 20030105190 A, Diehl
- US 3848594 A

- US 4662875 A
- US 4846815 A
- US 4894060 A
- US 4946527 A
- US 6432098 B
- US 4963140 A, Robertson
- US 4699622 A
- US 5242436 A
- US 5499978 A
- US 5507736 A
- US 5591152 A
- US 4515595 A
- US 4710189 A

**Non-patent literature cited in the description**

- Contact angle, wettability and adhesion. American Chemical Society, 1964